# EUROPEAN PATENT SPECIFICATION

(11) **EP 4 316 438 B1**
(45) Date of publication and mention of the grant of the patent: **19.11.2025**
(21) Application number: 23189389.2
(22) Date of filing: 03.08.2023
(51) Int. Cl.: A61F 13/15

(54) **MACHINE FOR MAKING ABSORBENT SANITARY ARTICLES**
MASCHINE ZUR HERSTELLUNG VON ABSORBIERENDEN HYGIENEARTIKELN
MACHINE POUR LA FABRICATION D'ARTICLES SANITAIRES ABSORBANTS

(30) Priority: 04.08.2022 IT 202200016608
(43) Date of publication of application: 07.02.2024
(73) Proprietor: GDM S.p.A., 40133 Bologna (IT)
(72) Inventor: ZAVALLONI, Alessandro, 40133 Bologna (IT); PIANTONI, Matteo, 40133 Bologna (IT); SACCOMANI, Alessandro, 40133 Bologna (IT); FRIGENI, Andrea, 40133 Bologna (IT)
(74) Representative: Casadei, Barbara

(56) References cited:
- EP-A2- 2 581 068

## Description

This invention relates to a machine for making absorbent sanitary articles.

In particular, a machine for making absorbent sanitary articles comprises at least one cutting unit for cutting a continuous web into segments. Document EP2581068 represents relevant prior art.

By way of example and without limiting the invention, the continuous web may be in the form of a web of non-woven fabric or a web comprising a polymeric material (for example, polyethylene or polypropylene) or a web comprising a natural or seminatural polymeric material (for example, cellulose and derivatives). Typically, the cutting unit comprises a cutting member and an anvil member acting in conjunction with each other.

The anvil member supports and feeds the continuous web which is being divided into segments by the cutting member.

Generally speaking, the cutting member is of a rotary type and comprises one or more cutting edges which impact the anvil member so that the continuous web supported by the anvil member is divided into segments.

The anvil member may also be of a rotary type and, in use, has a constant rotation speed.

In order to make a discrete segment of length equal to the size of the absorbent article to be made, the cutting member must make two suitably spaced, consecutive cuts and, therefore, during cutting, the cutting edge must move at the same rotation speed as the anvil member.

After making the cut, the law of motion of the cutting member may vary, meaning that it may accelerate and then decelerate when the cutting edge again needs to move at the same speed as the anvil member, or vice versa. Nowadays, the cutting member is driven by an electric actuator and an electronic cam controls the speed of the cutting member according to a law of motion corresponding to a respective size of the absorbent article.

The electronic cam allows applying two or more laws of motion to the cutting member so that a single cutting unit can be used to make segments corresponding to two or more sizes of absorbent articles.

This has undoubted advantages in terms of reducing machine down times for size changeover operations.

Flexibility is not the only requirement to be met by current machines, which must also be able to operate at high production speeds.

This affects the choice of actuator for the cutting member which, because it has to follow the laws of motion applied by the electronic cam to range from a small size, denoted "S", to a large size, denoted "L", must necessarily be high-powered, thus involving high costs and a significant installation footprint.

It must therefore be borne in mind that the performance required of the machine in terms of production speed clashes with the need for flexibility.

Another solution adopted to meet the need for flexibility without having to reduce production speed is to provide the machine with two cutting units, each having a respective anvil member and cutting member, so as to be able to activate one or the other, depending on the size of the absorbent article to be made.

This solution not only has a higher cost than that with the single cutting unit but also involves machine down time when changing over from one cutting unit to the other.

Moreover, providing the machine with a plurality of cutting units, each having a respective anvil member and cutting member, inevitably increases the cost of constructional parts, including redundant parts, and of the drives needed for them.

The Applicant has developed a machine for making absorbent sanitary articles, comprising at least one cutting unit configured to divide a continuous web into segments intended for manufacturing an absorbent sanitary article.

The cutting unit comprises an anvil member and a cutting member acting in conjunction with the anvil member.

The anvil member comprises a drum, rotating about a respective axis of rotation, for retaining the continuous web and the segments obtained therefrom.

The cutting unit comprises a control and drive unit configured to control the movement of the cutting member at least as a function of the rotation speed of the drum of the anvil member.

The cutting member comprises a first cutting roller and a second cutting roller, distinct from each other, both acting in conjunction with the drum of the anvil member and each rotating about a respective axis of rotation. Actuating means are configured to set the first cutting roller in rotation about its axis of rotation via respective motion transmission means and to set the second cutting roller in rotation about its axis of rotation via respective motion transmission means.

The control and drive unit is configured to selectively command the actuation of the first cutting roller and the second cutting roller via the actuating means as a function of a first size range and a second size range of absorbent sanitary articles respectively, and to apply to the first cutting roller a law of motion corresponding to a respective size belonging to the first size range and to apply to the second cutting roller a law of motion corresponding to a respective size belonging to the second size range.

Each law of motion has respective accelerations and decelerations.

Advantageously, the possibility of dividing the laws of motion of the cutting member between two distinct cutting rollers allows meeting the need for flexibility without negatively impacting the production speed and thus improving the machine efficiency compared to the prior art solutions.

Another advantage is due to the limited size of the transmission systems, which in turn translates as lower costs, lower consumption and smaller footprint compared to the prior art solutions.

Advantageously, moreover, changing over to a different size does not require any additional operation.

Advantageously, each cutting rollers corresponds to specific laws of motion, thereby maximizing performance in terms of the speed of each roller.

Advantageously, once a size range processable by a cutting unit of known type has been defined, partializing this range into two or more ranges allows increasing production speeds without increasing the power of the drive systems.

Preferably, the control and drive unit is configured to be programmed from the outside and to receive instructions relating to the laws of motion of the actuating means.

Advantageously, the settings of the control and drive unit are based on the sizes of the absorbent article to be processed.

Preferably, the cutting unit comprises first actuating means for actuating the first cutting roller and second actuating means for actuating the second cutting roller.

Advantageously, by dedicating a respective actuator to each cutting roller maximizes performance in terms of achievable production speeds and minimizes size changeover times.

Alternatively, the actuating means are configured to move either the first cutting roller or the second cutting roller. Advantageously, it is possible to use a single actuator, to be connected to the first cutting roller or to the second cutting roller based on the sizes to be processed.

Preferably, the actuating means comprise first actuating means for actuating the first cutting roller and second actuating means for actuating the second cutting roller.

This invention provides a method for making absorbent sanitary articles, comprising a step of cutting to divide a continuous web into segments intended for manufacturing an absorbent sanitary article.

The step of cutting comprises selectively commanding the rotation of one between a first cutting roller and a second cutting roller, both acting in conjunction with the same anvil member, as a function of a respective first size range and of a second size range of absorbent sanitary articles, and applying the rotation speed to one between the first cutting roller or the second cutting roller.

In the step of cutting, while one between the first cutting roller and the second cutting roller rotates about a respective axis of rotation, the other remains in a paused condition.

The step of cutting comprises applying a law of motion to the rotation of the first cutting roller or of the second cutting roller corresponding to a respective size belonging to the first size range or to the second size range, each law of motion having respective accelerations and decelerations.

Further features and advantages of the invention are more apparent in the exemplary, hence non-limiting description which follows of a preferred but non-exclusive embodiment of a machine for making absorbent sanitary articles and of the related method.

The description is set out below with reference to the accompanying drawings which are provided solely for purposes of illustration without restricting the scope of the invention and in which:

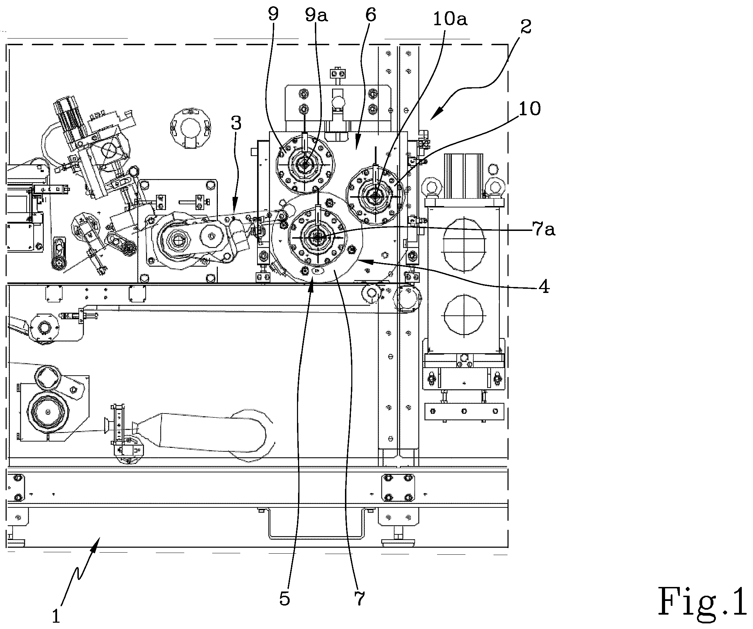

- Figure 1 is a schematic front view illustrating a machine for making absorbent sanitary articles according to this invention;

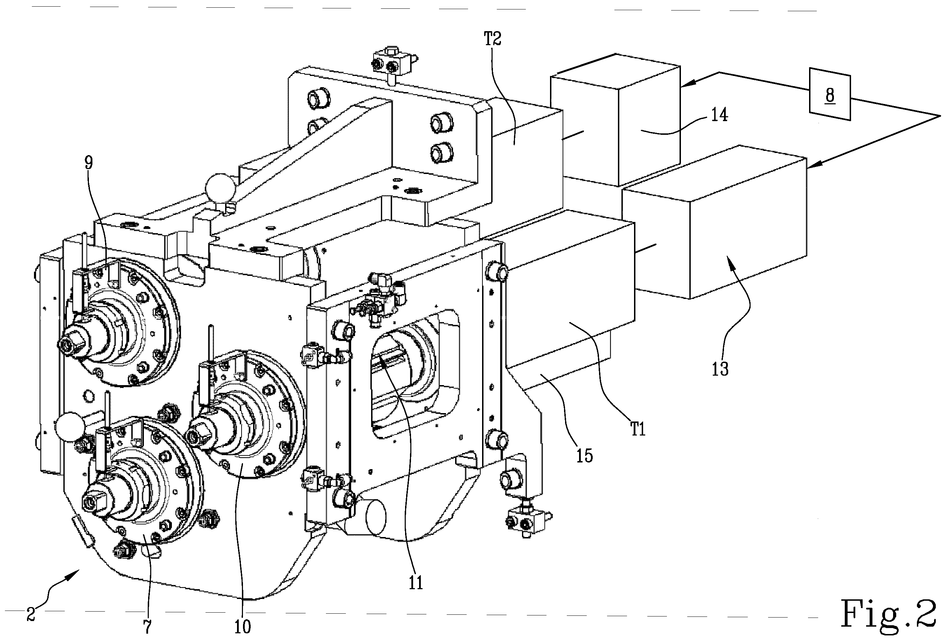

- Figure 2 is a schematic perspective view of a cutting unit of the machine of Figure 1.

The reference numeral 1 denotes a machine for making absorbent sanitary articles according to this invention.

The machine 1 comprises at least one cutting unit 2 configured to divide a continuous web 3 into segments 4 intended for manufacturing an absorbent sanitary article.

The cutting unit 2 comprises an anvil member 5 and a cutting member 6 acting in conjunction with the anvil member 5.

The anvil member 5 comprises a drum 7, rotating about a respective axis of rotation 7a, for retaining the continuous web 3 and the segments 4 obtained therefrom.

The cutting unit 2 comprises a control and drive unit 8 configured to control the movement of the cutting member 6 at least as a function of the rotation speed of the drum 7 of the anvil member 5.

The cutting member 2 comprises a first cutting roller 9 and a second cutting roller 10, both acting in conjunction with the drum 7 of the anvil member 5 and each rotating about a respective axis of rotation 9a, 10a.

The first cutting roller 9 and the second cutting roller 10 each comprise respective cutting members **11** driven in rotation about respective axes of rotation 9a, 10a.

The control and drive unit 8 is configured to control the rotation speed of the first cutting roller 9 or, alternatively, the rotation speed of the second cutting roller 10 in relation to a respective size of an absorbent article to be made. The control and drive unit 8 is configured to control the rotation speed of the first roller 9 as a function of a first size range or, alternatively, to control the rotation speed of the second roller 10 as a function of a second size range. Advantageously, the control and drive unit 8 is configured to control the rotation speed of the first cutting roller 9 or, alternatively, the rotation speed of the second cutting roller 10 according to the size to be processed.

Thus, the first cutting roller 9 and the second roller 10 can rotate at the maximum speeds permitted by the respective actuators since they are controlled by respective laws of motion corresponding to the sizes associated with the first roller 9 and the second roller 10.

The control and drive unit 8 is configured to control the rotation speed of the first roller 9 or of the second roller 10 according to a law of motion corresponding to a respective size belonging to the first size range or to the second size range; each law of motion has respective accelerations and decelerations.

The control and drive unit 8 is configured to receive instructions relating to the laws of motion to be applied to the first roller 9 and to receive instructions relating to the laws of motion to be applied to the second roller 10.

The control and drive unit 8 is configured to be programmed from the outside and to receive instructions relating to the laws of motion of the actuating means 13, 14.

For example, the instructions may be entered using a control panel installed in the machine 1.

In a first variant embodiment, the machine 1 comprises first actuating means 14 for actuating the first cutting roller 9 and second actuating means 13 for actuating the second cutting roller 10.

Preferably, the actuators 13, 14 are electric actuators.

In a second variant embodiment, the machine 1 comprises actuating means 14, 13 configured to move the first cutting roller 9 or, alternatively, the second cutting roller 10.

In this embodiment, if the roller being moved is the first cutting roller 9 to make the sizes corresponding to the first size range, the actuating means 14 are associated with the first cutting roller 9.

If the roller being moved is the second cutting roller 10 to make the sizes corresponding to the second size range, the actuating means 14 are associated with the second cutting roller 10.

The actuating means 13, 14 are configured to set the first cutting roller 9 in rotation about its axis of rotation 9a via respective motion transmission means T1 and to set the second cutting roller 10 in rotation about its axis of rotation 10a via respective motion transmission means T2.

The control and drive unit 8 is configured to selectively command the actuation of the first cutting roller 9 and the second cutting roller 10 via the actuating means 13, 14 as a function of a first size range and a second size range of absorbent sanitary articles respectively, and to apply to the first cutting roller 9 a law of motion corresponding to a respective size belonging to the first size range and to impart to the second cutting roller 10 a law of motion corresponding to a respective size belonging to the second size range; each law of motion has respective accelerations and decelerations. The machine 1 comprises actuating means 15 for actuating the drum 7 for retaining the continuous web 3 and the segments 4 obtained therefrom.

Preferably, the actuating means mentioned in this disclosure are electric actuators.

This invention also relates to a method for making absorbent sanitary articles, comprising a step of cutting configured to divide a continuous web 3 into segments 4 intended for manufacturing an absorbent sanitary article. The step of cutting comprises controlling the rotation speed of a first cutting roller 9 or of a second cutting roller 10, both acting in conjunction with the same anvil member 5, as a function of a respective first size range and of a second size range of absorbent sanitary articles.

The step of cutting comprises selectively commanding the rotation of one between a first cutting roller 9 and a second cutting roller 10, both acting in conjunction with the same anvil member 5, as a function of a respective first size range and of a second size range of absorbent sanitary articles, and applying the rotation speed to one between the first cutting roller 9 or the second cutting roller 10.

In the step of cutting, while one between the first cutting roller 9 and the second cutting roller 10 rotates about a respective axis of rotation 9a, 10a, the other remains in a paused condition.

The step of cutting comprises applying a law of motion to the rotation of the first cutting roller 9 or of the second cutting roller 10 corresponding to a respective size belonging to the first size range or to the second size range, each law of motion having respective accelerations and decelerations.

## Claims

1. A machine for making absorbent sanitary articles, comprising at least one cutting unit (2) configured to divide a continuous web (3) into segments (4) intended for manufacturing an absorbent sanitary article;
the cutting unit (2) comprises an anvil member (5) and a cutting member (6) acting in conjunction with the anvil member (5);
the anvil member (5) comprises a drum (7), rotating about a respective axis of rotation (7a), for retaining the continuous web (3) and the segments (4) obtained therefrom;
the cutting unit (2) comprises a control and drive unit (8) configured to control the movement of the cutting member (6) at least as a function of the rotation speed of the drum (7) of the anvil member (5);
**characterized in that**
the cutting member (6) comprises a first cutting roller (9) and a second cutting roller (10), distinct from each other, both acting in conjunction with the drum (7) of the anvil member (5) and each rotating about a respective axis of rotation (9a, 10a);
actuating means (13, 14) are configured to set the first cutting roller (9) in rotation about its axis of rotation (9a) via respective motion transmission means (T1) and to set the second cutting roller (10) in rotation about its axis of rotation (10a) via respective motion transmission means (T2);
the control and drive unit (8) is configured to selectively command the actuation of the first cutting roller (9) and the second cutting roller (10) via the actuating means (13, 14) as a function of a first size range and a second size range of absorbent sanitary articles respectively, and to impart to the first cutting roller (9) via the actuating means (13, 14) a law of motion corresponding to a respective size belonging to the first size range and to apply to the second cutting roller (10) via the actuating means (13, 14) of a law of motion corresponding to a respective size belonging to the second size range; each law of motion has respective accelerations and decelerations.

2. The machine according to the preceding claim, wherein the control and drive unit (8) is configured to be programmed from the outside and to receive instructions relating to the laws of motion of the actuating means (13, 14).

3. The machine according to any one of the preceding claims, wherein the actuating means (14) are configured to move the first cutting roller (9) or, alternatively, the second cutting roller (10).

4. The machine according to any one of the preceding claims, wherein the actuating means (13, 14) comprise first actuating means (14) for actuating the first cutting roller (9) and second actuating means (13) for actuating the second cutting roller (10).

5. A method for making absorbent sanitary articles, comprising a step of cutting to divide a continuous web (3) into segments (4) intended for manufacturing an absorbent sanitary article;
the step of cutting comprises selectively commanding the rotation of one between a first cutting roller (9) and a second cutting roller (10), both acting in conjunction with the same anvil member (5), as a function of a respective first size range and of a second size range of absorbent sanitary articles, and applying the rotation speed to one between the first cutting roller (9) or the second cutting roller (10);
in the step of cutting, while one between the first cutting roller (9) and the second cutting roller (10) rotates about a respective axis of rotation (9a, 10a), the other remains in a paused condition;
the step of cutting comprises applying a law of motion to the rotation of the first cutting roller (9) or of the second cutting roller (10) corresponding to a respective size belonging to the first size range or to the second size range, each law of motion having respective accelerations and decelerations.

## Patentansprüche

1. Maschine zur Herstellung von absorbierenden Hygieneartikeln, umfassend mindestens eine Schneideinheit (2), die ausgelegt ist, um eine durchgehende Bahn (3) in Segmente (4) zu teilen, die zum Fertigen eines absorbierenden Hygieneartikels bestimmt sind,
wobei die Schneideinheit (2) ein Auflageelement (5) und ein Schneidelement (6), das in Verbindung mit dem Auflageelement (5) wirkt, umfasst,
wobei das Auflageelement (5) eine Trommel (7) umfasst, die sich um eine jeweilige Rotationsachse (7a) dreht, um das durchgehende Band (3) und die daraus gewonnenen Segmente (4) zu halten,
wobei die Schneideinheit (2) eine Steuer- und Antriebseinheit (8) umfasst, die ausgelegt ist, um die Bewegung des Schneidelements (6) zumindest abhängig von der Drehzahl der Trommel (7) des Auflageelements (5) zu regeln,
**dadurch gekennzeichnet, dass**
das Schneidelement (6) eine erste Schneidwalze (9) und eine zweite Schneidwalze (10) umfasst, die voneinander verschieden sind und beide in Verbindung mit der Trommel (7) des Auflageelements (5) wirken und sich jeweils um eine jeweilige Rotationsachse (9a, 10a) drehen;
Betätigungsmittel (13, 14) ausgelegt sind, um die erste Schneidwalze (9) um ihre Rotationsachse (9a) über jeweilige Bewegungsübertragungsmittel (T1) in Drehung zu versetzen und die zweite Schneidwalze (10) um ihre Rotationsachse (10a) über jeweilige Bewegungsübertragungsmittel (T2) in Drehung zu versetzen;
die Steuer- und Antriebseinheit (8) ausgelegt ist, um die Betätigung der ersten Schneidwalze (9) und der zweiten Schneidwalze (10) über die Betätigungsmittel (13, 14) abhängig jeweils von einer ersten Größenordnung und einer zweiten Größenordnung absorbierender Hygieneartikel selektiv zu steuern und auf die erste Schneidwalze (9) über die Betätigungsmittel (13, 14) ein Bewegungsprinzip entsprechend einer jeweiligen Größe, die der ersten Größenordnung angehört, zu übertragen, und auf die zweite Schneidwalze (10) über die Betätigungsmittel (13, 14) ein Bewegungsprinzip entsprechend einer jeweiligen Größe, die der zweiten Größenordnung angehört, anzuwenden, wobei ein jedes Bewegungsprinzip jeweilige Beschleunigungen und Verzögerungen aufweist.

2. Maschine nach dem vorhergehenden Anspruch, wobei die Steuer- und Antriebseinheit (8) ausgelegt ist, um von der Außenseite programmiert zu werden und Anweisungen in Bezug auf die Bewegungsprinzipien der Betätigungsmittel (13, 14) zu empfangen.

3. Maschine nach einem der vorhergehenden Ansprüche, wobei die Betätigungsmittel (14) ausgelegt sind, um die erste Schneidwalze (9) oder alternativ die zweite Schneidwalze (10) zu bewegen.

4. Maschine nach einem der vorhergehenden Ansprüche, wobei die Betätigungsmittel (13, 14) erste Betätigungsmittel (14) zum Betätigen der ersten Schneidwalze (9) und zweite Betätigungsmittel (13) zum Betätigen der zweiten Schneidwalze (10) umfassen.

5. Verfahren zur Herstellung von absorbierenden Hygieneartikeln, umfassend einen Schritt zum Schneiden, um eine durchgehende Bahn (3) in Segmente (4) zu teilen, die zum Fertigen eines absorbierenden Hygieneartikels bestimmt sind,
wobei der Schritt zum Schneiden das selektive Steuern der Drehung entweder einer ersten Schneidwalze (9) oder einer zweiten Schneidwalze (10), die beide in Verbindung mit demselben Auflageelement (5) wirken, abhängig von einer jeweiligen ersten Größenordnung und einer zweiten Größenordnung von absorbierenden Hygieneartikeln und das Anwenden der Drehzahl entweder auf die erste Schneidwalze (9) oder die zweite Schneidwalze (10) umfasst,
wobei beim Schritt zum Schneiden, während sich entweder die erste Schneidwalze (9) oder die zweite Schneidwalze (10) um eine jeweilige Rotationsachse (9a, 10a) dreht, die andere Walze in einem pausierten Zustand bleibt,
wobei der Schritt zum Schneiden das Anwenden eines Bewegungsprinzips auf die Drehung der ersten Schneidwalze (9) oder der zweiten Schneidwalze (10) entsprechend einer jeweiligen Größe, die der ersten Größenordnung oder der zweiten Größenordnung angehört, umfasst, wobei ein jedes Bewegungsprinzip jeweilige Beschleunigungen und Verzögerungen aufweist.

## Revendications

1. Machine pour la fabrication d'articles sanitaires absorbants, comprenant au moins une unité de coupe (2) configurée pour diviser une bande continue (3) en segments (4) destinés à fabriquer un article sanitaire absorbant ;
l'unité de coupe (2) comprend un élément enclume (5) et un élément de coupe (6) agissant conjointement avec l'élément enclume (5) ;
l'élément enclume (5) comprend un tambour (7), tournant autour d'un axe de rotation (7a) respectif, pour retenir la bande continue (3) et les segments (4) obtenus à partir de celle-ci ;
l'unité de coupe (2) comprend une unité de commande et d'entraînement (8) configurée pour commander le mouvement de l'élément de coupe (6) au moins en fonction de la vitesse de rotation du tambour (7) de l'élément enclume (5) ;
**caractérisée en ce que**
l'élément de coupe (6) comprend un premier rouleau de coupe (9) et un second rouleau de coupe (10), distincts l'un de l'autre, agissant tous deux conjointement avec le tambour (7) de l'élément enclume (5) et tournant chacun autour d'un axe de rotation (9a, 10a) respectif ; des moyens d'actionnement (13, 14) sont configurés pour mettre le premier rouleau de coupe (9) en rotation autour de son axe de rotation (9a) par le biais de moyens de transmission de mouvement (T1) respectifs et pour mettre le second rouleau de coupe (10) en rotation autour de son axe de rotation (10a) par le biais de moyens de transmission de mouvement (T2) respectifs ;
l'unité de commande et d'entraînement (8) est configurée pour commander sélectivement l'actionnement du premier rouleau de coupe (9) et du second rouleau de coupe (10) par le biais des moyens d'actionnement (13, 14) en fonction d'une première gamme de tailles et d'une seconde gamme de tailles d'articles sanitaires absorbants respectivement, et pour communiquer au premier rouleau de coupe (9) par le biais des moyens d'actionnement (13, 14) une loi de mouvement correspondant à une taille respective appartenant à la première gamme de tailles et pour appliquer au second rouleau de coupe (10) par le biais des moyens d'actionnement (13, 14) une loi de mouvement correspondant à une taille respective appartenant à la seconde gamme de tailles ; chaque loi de mouvement a des accélérations et des décélérations respectives.

2. Machine selon la revendication précédente, dans laquelle l'unité de commande et d'entraînement (8) est configurée pour être programmée depuis l'extérieur et pour recevoir des instructions relatives aux lois de mouvement des moyens d'actionnement (13, 14).

3. Machine selon l'une quelconque des revendications précédentes, dans laquelle les moyens d'actionnement (14) sont configurés pour déplacer le premier rouleau de coupe (9) ou, alternativement, le second rouleau de coupe (10) .

4. Machine selon l'une quelconque des revendications précédentes, dans laquelle les moyens d'actionnement (13, 14) comprennent des premiers moyens d'actionnement (14) pour actionner le premier rouleau de coupe (9) et des seconds moyens d'actionnement (13) pour actionner le second rouleau de coupe (10).

5. Procédé pour la fabrication d'articles sanitaires absorbants, comprenant une étape de coupe pour diviser une bande continue (3) en segments (4) destinés à la fabrication d'un article sanitaire absorbant ;
l'étape de coupe comprend la commande sélective de la rotation de l'un entre un premier rouleau de coupe (9) et un second rouleau de coupe (10), tous deux agissant conjointement avec le même élément enclume (5), en fonction d'une première plage de taille respective et d'une seconde plage de taille d'articles sanitaires absorbants, et l'application de la vitesse de rotation à l'un entre le premier rouleau de coupe (9) ou le second rouleau de coupe (10) ;
dans l'étape de coupe, tandis que l'un entre le premier rouleau de coupe (9) et le second rouleau de coupe (10) tourne autour d'un axe de rotation (9a, 10a) respectif, l'autre reste dans un état en pause ;
l'étape de coupe comprend l'application d'une loi de mouvement à la rotation du premier rouleau de coupe (9) ou du second rouleau de coupe (10) correspondant à une taille respective appartenant à la première gamme de tailles ou à la seconde gamme de tailles, chaque loi de mouvement ayant des accélérations et des décélérations respectives.
